# EUROPEAN PATENT APPLICATION

(11) **EP 4 620 529 A2**
(43) Date of publication of application: **24.09.2025**
(21) Application number: 25195704.9
(22) Date of filing: 13.04.2022
(51) Int. Cl.: A61P 31/14

(54) **COMPOSITION COMPRISING A EUKARYOTIC MICROALGAE OR AN EXTRACT THEREOF FOR USE IN THE TREATMENT OF A VIRAL DISEASE**

(30) Priority: 13.04.2021 EP 21168138
(62) Divisional of application: 22722768.3
(71) Applicant: Philip Morris Products, S.A., 2000 Neuchâtel (CH)
(72) Inventor: ASTROLOGO, Letizia, 2000 Neuchâtel (CH); HOENG, Julia, 2000 Neuchâtel (CH); RENGGLI, Kasper, 2000 Neuchâtel (CH); VAN DER TOORN, Marco, 2000 Neuchâtel (CH)
(74) Representative: Reddie & Grose LLP

(57) **Abstract**

A composition comprising a eukaryotic microalgae or an extract thereof may be for use in the treatment of a viral disease, e.g. coronavirus, and for use as an inhibitor of the binding of Spike protein receptor binding domain (RBD) to Angiotensin-Converting Enzyme 2 (ACE2). A compound derivable from eukaryotic microalgae may also be for use in the treatment of a viral disease. In turn, a eukaryotic microalgae may be used as an antiviral agent.

## Description

The invention relates to a composition comprising a eukaryotic microalgae or an extract thereof. More specifically, the invention relates to a composition comprising a eukaryotic microalgae or an extract thereof for use in the treatment of a viral disease, a compound derivable from eukaryotic microalgae for use in the treatment of a viral disease, a composition comprising a eukaryotic microalgae or an extract thereof for use as an inhibitor of the binding of the Spike protein receptor binding domain (RBD) to Angiotensin-Converting Enzyme 2 (ACE2) and use of a eukaryotic microalgae or an extract thereof as an antiviral agent.

### Background

A virus is a small infectious agent that replicates only inside living cells of other organisms. Viruses can infect various life forms, from animals and plants to microorganisms, including bacteria and archaea. Virus infection typically causes disease symptoms in the affected subject, which can vary from mild to severe symptoms, which can result in morbidity and mortality.

Some of the most common symptoms associated with viral infection comes from human respiratory diseases. Examples of human respiratory diseases resulting from viruses include influenza, which is caused by various influenza viruses, severe acute respiratory syndrome (SARS), which is caused by coronavirus (SARS-CoV), illness of the respiratory system, which can be caused by adenoviruses, and bronchitis and child pneumonia, which is caused by the respiratory syncytial virus (RSV).

Coronavirus disease 2019 commonly known as "COVID19", is an infectious disease caused by the severe acute respiratory syndrome coronavirus 2 (SARS-CoV-2). The COVID-19 pandemic, also known as the coronavirus pandemic, is an ongoing pandemic with more than 88 million confirmed cases and more than 1.89 million deaths worldwide as of March 2021. Mortality is highest with elderly people and others with underlying health problems. While most people develop mild symptoms, some develop severe symptoms like pneumonia, respiratory failure, shock, or multi-organ dysfunction.

The high mortality of patients admitted to critical care with severe acute SARS-CoV-2 infection has been attributed to a combination of hyper-inflammatory syndromes, coagulation disorders, and pathological activation of immunological pathways leading to a cytokine release syndrome.

There is an urgent need for effective, early treatments that have both antiviral and disease-modifying actions to limit the pressure that severe COVID-19 cases place on healthcare infrastructure. While vaccination is the most efficacious route for primary prevention of SARS-CoV-2 infection, intranasal or intra-oral delivery of antiviral agents may be an important additional approach for preventing infection and the spread of SARS-CoV-2 and other respiratory viruses.

Typically, SARS-CoV-2 spreads mainly through exposure to aerosol from infected people as they breathe, cough, sneeze, or speak. Thus, one of the main entry points of the SARS-CoV-2 virus into the human body is via the mouth, nose or eyes. Being able to pharmacologically block these entry points could help to reduce the number of cases of COVID-19. When it enters a subject via their mouth, nose or eyes, the virus replicates by attaching to the host cell surface before entering the cell. The Spike protein receptor binding domain (RBD) recognizes and attaches to the Angiotensin-Converting Enzyme 2 (ACE2) receptor found on the surface of type I and II pneumocytes, endothelial cells, and ciliated bronchial epithelial cells. ACE2 is an exopeptidase that catalyses the conversion of angiotensin II to angiotensin 1-7 and L-phenylalanine. Angiotensin II is part of the classical renin angiotensin system (RAS), a hormone system that regulates fluid balance, blood pressure and maintains vascular tone. ACE2 has also been proven to be the main receptor for the entry of respiratory viruses like human respiratory coronavirus NL63, SARS-coronavirus (SARS-CoV), and other SARS-CoV-2 variants like the new B117. The binding of SARS-CoV and SARS-CoV-2 to ACE2, followed by membrane fusion and virus entry into the cell, has been linked to downregulation of ACE2 receptors. This downregulation of ACE2 also impairs its pivotal protective function, the degradation of angiotensin II to angiotensin 1-7. Angiotensin II is associated with a variety of adverse health outcomes, including cardiovascular disease, enhanced inflammation in response to oxidative stress, and hyper-coagulation. Furthermore, angiotensin II is also a macrophage activator that leads to increased secretion of IL-6 and TNFα as well as other inflammatory reactions. ACE2 receptors counteract angiotensin II by degrading, and thereby eliminating, it and also by catalysing its conversion to angiotensin 1-7, resulting in several positive and even counter-regulatory actions against angiotensin II. Although selective ACE2 inhibitors, such as MLN-4760, exist that might inhibit the cellular attachment of a virus, such as SARS-CoV-2, they would also block the degradation of angiotensin II to angiotensin 1-7.

Therefore, a need exists for a new therapeutic mode of action that blocks viral entry via the SARS-CoV-2 RBD without inhibiting ACE2.

### Summary of Invention

The present invention provides a composition comprising a eukaryotic microalgae or an extract thereof for use in the treatment of a viral disease.

As used herein the term "treatment" may include preventative (prophylactic) treatment; curative treatment; maintenance treatment, and promotive treatment.

It has been surprisingly found that a composition comprising a eukaryotic microalgae or an extract thereof has beneficial effects in preventing the spread of a viral disease, such as, COVID-19.

The viral disease may be a respiratory viral disease. The viral disease may be caused by one or more of the following: influenza virus, respiratory syncytial virus (RSV), parainfluenza virus, or respiratory adenovirus. The viral disease may be Severe acute respiratory syndrome (SARS). The viral disease may be MERS and/or COVID-19. The viral disease may be caused by a SARS-associated virus. The viral disease may be caused by coronavirus (SARS-CoV-2). Herein, coronavirus is understood to include the subfamily *Orthocoronavirinae* in the family *Coronaviridae.*

More specifically, it has been found that a composition comprising a eukaryotic microalgae or an extract thereof may inhibit the viral Spike protein receptor binding domain (RBD) binding to the Angiotensin-Converting Enzyme 2 (ACE2) receptor. Advantageously, the composition comprising a eukaryotic microalgae or an extract thereof does not affect ACE2 activity. In turn, the composition comprising a eukaryotic microalgae or an extract thereof does not compromise the functioning of the respiratory system or lead to cardiovascular disease, enhanced inflammation in response to oxidative stress, or hyper-coagulation. Advantageously, the composition prevents a viral disease from reaching the brain or the bronchi and lungs. Thus, the composition of the present invention may be used to inhibit or prevent the respiratory system from being compromised. Further, by preventing the colonization of the viral infection of olfactory and gingival epithelial cells, from where the viral infection can migrate to the central nervous system in order to attack the respiratory centre and affect neurons, the composition of the invention may also prevent SARS-CoV-2 infections from causing the loss of smell.

In an alternative embodiment, the invention may relate to a prokaryotic microalgae or an extract thereof. Aspects of the invention described in relation to eukaryotic microalgae or an extract thereof may be equally applicable to the prokaryotic microalgae or an extract thereof of the invention. In particular, the invention may relate to a composition comprising a prokaryotic microalgae or an extract thereof for use in the treatment of a viral disease. The prokaryotic microalgae may include cyanobacteria, (formerly called "blue-green algae").

The term "eukaryotic microalgae" is understood to mean the diverse group of aquatic organisms that have the ability to conduct photosynthesis. Eukaryotic microalgae are unicellular species which exist individually, or in chains or groups. The eukaryotic microalgae may include green, brown and red algae. More specifically, eukaryotic microalgae may include the phyla: *Rhodophyta* (red algae), *Chlorophyta* (green algae), and *Phaeophyta* (brown algae). The eukaryotic microalgae may be of the genus *Nannochloropsis* or *Tetraselmis.*

Preferably, the eukaryotic microalgae may be a green eukaryotic microalgae, for example, green eukaryotic microalgae which is capable of photosynthesis. The eukaryotic microalgae may be *Chlorophyta,* preferably Chlorella. The term "Chlorella" is understood to relate to the genus of single-cell, eukaryotic, green algae belonging to the division of the *Chlorophyta.* Advantageously, Chlorella is a Generally Recognized as Safe (GRAS) substance by the FDA.

Preferably, the eukaryotic microalgae may be selected from one or more of *Chlorella autotrophica, Chlorella colonials, Chlorella lewinii, Chlorella minutissima, Chlorella pituita, Chlorella pulchelloides, Chlorella pyrenoidosa, Chlorella rotunda, Chlorella singularis, Chlorella sorokiniana, Chlorella variabilis, Chlorella volutis, Chlorella vulgaris, Tetraselmis Chuii, Nannochloropsis gaditana.*

More preferably, the composition may comprise a eukaryotic microalgae or an extract thereof for use in the treatment of a viral disease caused by coronavirus SARS-CoV-2, wherein the eukaryotic microalgae is *Chlorella.*

It has been surprisingly found that a composition comprising Chlorella or an extract thereof may have beneficial effects in the treatment of COVID-19. More specifically, it has been found that Chlorella may inhibit viral RBD binding to the ACE2 receptor without affecting ACE2 activity. Thus, a composition comprising Chlorella or an extract thereof may be used for the treatment of SARS-CoV-2 infection and COVID-19.

Furthermore, it has been found that Chlorella prevents viral binding to ACE2 receptors present on cells in the mouth, nose and/or eyes. By preventing the viral disease from binding to these cell surfaces, Chlorella inhibits the ability of the viral disease to enter and replicate in the cell and prevents further infection from viral disease particles released by cells that have already been infected.

The extract of the eukaryotic microalgae may comprise one or more of the following: protein, lipid, vitamin, mineral, folate, iron, fibre, and/or pigment. The vitamin may include vitamin B and D12.

The protein may be selected from one or more of Plastocyanin (A0A2P6TGD1), Glycine-rich 2 (A0A2P6TDY2), SMAD FHA domain-containing (A0A2P6U3K7), Thylakoid lumenal kDa chloroplastic (A0A2P6TNZ5), Peptidylprolyl isomerase (A0A2P6TGB0), Calmodulin (A0A2P6TFR8), 10 kDa chaperonin-like (A0A2P6THP7), Membrane isoform A (A0A2P6TEF6), L-inducible nipa (A0A2P6TU12), Beta-lg-H3 fasciclin (A0A2P6TQQ9).

The pigment may be chlorophyll. The chlorophyll may be one or more of chlorophyll a, chlorophyll b, chlorophyll c1, chlorophyll c2, chlorophyll d, chlorophyll f. Preferably, the extract may comprise chlorophyll c1 and/or c2, which is prevalent in many algae. The extract may comprise a pre-cursor of chlorophyll, e.g. a pre-cursor derived from glutamate.

The extract of the eukaryotic microalgae may comprise natural chlorophyll or synthetic chlorophyll. Advantageously, synthetic chlorophyll - registered as a food additive colorant (E140) - is food-safe.

The composition comprising a eukaryotic microalgae or an extract thereof for use in the treatment of a viral disease may be administered locally. Preferably the composition according to the invention may be administered topically. The composition may be a powder; liquid; aerosol; gel; paste or cream. The composition may be administered to the nasal or oral cavity. Preferably the composition according to the invention may be administered as a nasal spray; a mouth wash; or an orally inhaled formulation. Local administration to the respiratory pathway is desirable as it helps maintain a high local concentration of the eukaryotic microalgae or extract thereof at the primary site of infection, e.g. airway respiratory epithelium, whilst keeping the systemic load low when compared to systemic administration. Advantageously, this provides a two-fold benefit: firstly, the composition administered as a nasal spray or mouth wash may stop viral infection via the nasal and oral cavities in a healthy subject by shielding the epithelium cells of the nasal and oral mucosa; and secondly, in the case of a newly diagnosed infected subject, the composition administered as a nasal spray or mouth wash may limit or prevent the viral particles released by the dying cells from colonizing new cells, such as the olfactory and gingival epithelium. Through limitation and/or prevention of further colonization, the virus is prevented from spreading to new pathways such as the cardiovascular system and/or prevented from infecting more cells of the respiratory epithelium or lower respiratory system.

The composition according to the invention may comprise at least about 0.001 µg/mL, at least about 0.01 µg/mL, at least about 0.1 µg/mL, at least about 1 µg/mL of a eukaryotic microalgae or an extract thereof.

The composition according to the invention may comprise no more than about 10000 µg/mL, no more than about 1000 µg/mL, no more than about 100 µg/mL, no more than about 10 µg/mL of a eukaryotic microalgae or an extract thereof.

The composition according to the invention may comprise about 0.001 µg/mL to about 10000 µg/mL, about 0.01 µg/mL to about 1000 µg/mL, about 0.1 µg/mL to about 100 µg/mL, about 1 to about 10 µg/mL of a eukaryotic microalgae or an extract thereof.

The composition comprising a eukaryotic microalgae or an extract thereof may comprise any range from the given endpoints. The composition comprising a eukaryotic microalgae or an extract thereof may enable effective doses of extract, e.g. for use in the treatment of a viral disease.

The invention also provides for a compound derivable from eukaryotic microalgae for use in the treatment of a viral disease. Herein, the expression "compound derivable from eukaryotic microalgae" is understood to mean that the compound may be isolated or extracted from microalgae, for example, the compound may be isolated or extracted from a culture of microalgae. Conventional methods for isolating or extracting compounds from microalgae are available to the skilled person, for example, in the form of commercial kits for isolating or extracting compounds from microalgae. The expression "compound derivable from eukaryotic microalgae" may also encompass chemically synthesised compounds which naturally appear in eukaryotic microalgae and thus may be "derivable" from eukaryotic microalgae by conventional methods.

The invention also provides for a composition comprising a eukaryotic microalgae or an extract thereof for use as an inhibitor of the binding of the Spike protein receptor binding domain (RBD) to Angiotensin-Converting Enzyme 2 (ACE2). Preferably, the composition comprising a eukaryotic microalgae or an extract thereof for use as an inhibitor of the binding of the RBD to ACE2 *in vivo.* For example, the composition comprising a eukaryotic microalgae or an extract thereof for use as an inhibitor of the binding of the RBD to ACE2 in a human subject.

Preferably, the composition comprising a eukaryotic microalgae or an extract thereof is for use as an inhibitor of the binding of the RBD to ACE2 without affecting ACE2 activity.

The invention provides for a composition comprising a eukaryotic microalgae or an extract thereof for use in the treatment of a viral disease and/or as an inhibitor of the binding of the Spike protein receptor binding domain (RBD) to Angiotensin-Converting Enzyme 2 (ACE2) providing a reduction in adverse events. Adverse events may include, but are not limited to, adverse events caused by an excess of angiotensin II such as, respiratory difficulties; cardiovascular events; inflammatory reactions, such as inflammation in response to oxidative stress; blood clots associated with thrombocytopenia; and hyper-coagulation, such as disseminated intravascular coagulation (DIC).

### List of Figures

Embodiments of the invention will now be described, by way of illustration only, with reference to the accompanying figures, in which:
Figure 1 is an image of a protein gel showing the Chlorella extract;
Figures 2A & 2B show the inhibition of ACE2:SARS-CoV-2 spike binding, measured by chemiluminescence using various concentrations of Chlorella extract [MIX0000036] (Fig 2A) and EDTA as positive control (Fig 2B);
Figure 3 shows the activity of ACE2 in the presence of Chlorella extract; and
Figure 4 shows the effect of Chlorella extract and Remdesivir on infection of Vero E6 cell culture with active SARS-CoV-2.

### Detailed description

### Experiment 1 - Proteomic characterization

The following experiments were performed with a Chlorella extract obtained from Algosource, Saint-Nazaire, France, which showed greatest homogeneity with *Chlorella sorokiniana* following proteomic analysis.

The proteome of the Chlorella extract was assessed by microfluidic electrophoresis (LabChip protein characterization system) and by liquid chromatography coupled to mass spectrometry (LC-MS/MS) in a data-dependent acquisition mode (protein profiling).

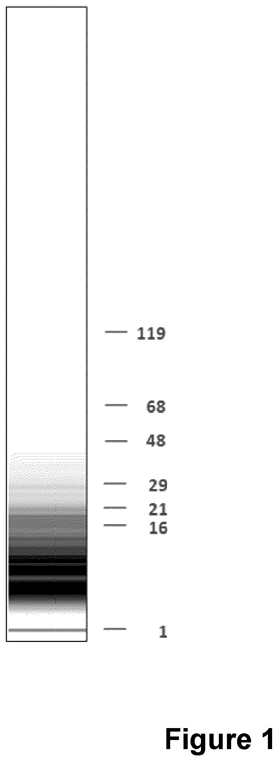

Microfluidic electrophoresis demonstrated the highest abundance of proteins in the low-molecular-weight range. Figure 1 is an image of a protein gel confirming that proteins below 20 kDa have the highest abundance.

Total protein abundance measurements by LC-MS using a data-dependent acquisition approach confirmed these observations (Table 1). Table 1 comprises a list of the most abundant proteins together with their accession number and normalized spectral abundance factor (NSAF) score. The spectral count analysis demonstrated that several proteins involved in photosynthesis are particularly abundant.

**Table 1: Total protein analysis of Chlorella extract**

| **Accession** | **Description** | **NSAF** |
|---|---|---|
| A0A2P6TGD1 | Plastocyanin OS=Chlorella sorokiniana OX=3076 GN=C2E21_7759 PE=3 SV=1 - [A0A2P6TGD1_CHLSO] | 0,019898485 |
| A0A2P6TDY2 | Glycine-rich 2 OS=Chlorella sorokiniana OX=3076 GN=C2E21_8624 PE=4 SV=1 - [A0A2P6TDY2_CHLSO] | 0,016135178 |
| A0A2P6U3K7 | SMAD FHA domain-containing OS=Chlorella sorokiniana OX=3076 GN=C2E21_0292 PE=4 SV=1 - [A0A2P6U3K7_CHLSO] | 0,01034577 |
| AOA2P6TNZ5 | Thylakoid lumenal kDa chloroplastic OS=Chlorella sorokiniana OX=3076 GN=C2E21_5696 PE=4 SV=1 - [A0A2P6TNZ5_CHLSO] | 0,008365729 |
| A0A2P6TGB0 | Peptidylprolyl isomerase OS=Chlorella sorokiniana OX=3076 GN=C2E21_7932 PE=4 SV=1 - [A0A2P6TGB0_CHLS0] | 0,007308394 |
| A0A2P6TFR8 | Calmodulin OS=Chlorella sorokiniana OX=3076 GN=C2E21_8040 PE=3 SV=1 - [A0A2P6TFR8_CHLSO] | 0,006574131 |
| A0A2P6THP7 | 10 kDa chaperonin-like OS=Chlorella sorokiniana OX=3076 GN=C2E21_7220 PE=3 SV=1 - [A0A2P6THP7_CHLSO] | 0,006500172 |
| A0A2P6TEF6 | Membrane isoform A OS=Chlorella sorokiniana OX=3076 GN=C2E21_8431 PE=4 SV=1 - [A0A2P6TEF6_CHLSO] | 0,006474275 |
| A0A2P6TU12 | L-inducible nipa OS=Chlorella sorokiniana OX=3076 GN=C2E21_3614 PE=4 SV=1 - [A0A2P6TU12_CHLSO] | 0,00596346 |
| A0A2P6TQQ9 | Beta-lg-H3 fasciclin OS=Chlorella sorokiniana OX=3076 GN=C2E21_4613 PE=4 SV=1 - [A0A2P6TQQ9_CHLSO] | 0,005914624 |

### Experiment 2 - Inhibitor screening assay

In line with the standard ACE2:SARS-CoV-2 Spike inhibitor screening assay protocol (BPSBioscience, San Diego, US) assay plates were coated with ACE2-His solution (1 µg/mL) for 1 h at room temperature (RT). ACE2-His coating solution was washed away in order to retain ACE2-His only coated to the assay plate. Assay plates were incubated for 10 min with blocking buffer to provide aspecific binding of Spike. After removing the blocking buffer, Chlorella extracts in various concentrations ranging from 0.001 to 100 µg/mL were incubated for 1 h followed by initiating the binding reaction using SARS-CoV-2 Spike (20 ng/well) solution. The mixtures were incubated for a further 1 h at RT and then washed and blocked again for 10 min. Finally, the plate was incubated with a secondary HRP-labelled antibody solution (1:1000) against the SARS-CoV-2 Spike for 1 h at RT. The HRP-labelled antibody was washed away and chemiluminescence was initiated and measured using an enhanced chemiluminescence substrate and a FLUOstar Omega plate reader (BMG Labtech).

In order to characterize the impact of the Chlorella extract on ACE2 : SARS-CoV-2 binding, a commercially available recombinant SARS-CoV-2 Spike inhibitor screening assay was used, which was designed for screening and profiling of inhibitors (BPSBioscience, San Diego, US).

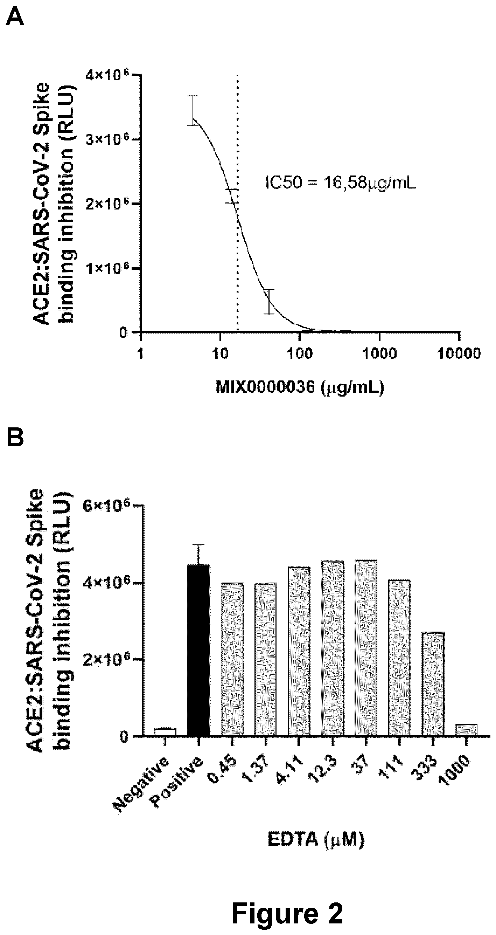

Figure 2A demonstrates that the Chlorella extract has a dose-dependent inhibition of the binding of ACE2 to SARS-CoV-2. In turn, this indicates that the main entry of the virus into the cell is blocked. More specifically, Figures 2A & 2B show the inhibition of ACE2:SARS-CoV-2 spike binding, measured by chemiluminescence using various concentrations of (Figure 2A, MIX0000036, Chlorella extract) and EDTA as positive control (Figure 2B).

ACE2:SARS-CoV-2 spike binding was measured in relative light units (RLU). The half maximal inhibitory concentration (IC50) was determined, which was IC50 = 16.58 µg/mL for the Chlorella extract. Figure 2B confirms that the assay as such was working. As negative control, no SARS-CoV-2 spike was applied (negative); as positive control 1 ng/µL SARS-CoV-2 spike was applied (positive). Ethylenediaminetetraacetic acid (EDTA) was used to confirm ACE2:SARS-CoV-2 spike binding at the tested range of 0.45 µM to 1000 µM.

### Experiment 3 - Non-interference with the catalytic pathway of ACE2

To characterize the impact of the Chlorella extract on ACE2 enzymatic activity, a cellular assay was used that monitors the catalytic activity of ACE2 with a fluorescent ACE2 substrate, in order to demonstrate successful inhibition by a loss of fluorescence.

The ACE2 enzymatic activity assay was designed to measure the exopeptidase activity of ACE2 and to screen for inhibition. Activity was measured using a commercially available synthetic fluorogenic substrate for ACE2 and rACE2 (R&D systems, Zug). rACE2 was used to generate a standard curve using the assay buffer, 75 mM TRIS, 1M NaCl, ph7.5. Natural extract screening was performed using 100 ng/mL rACE2 and 15 µM ACE2 fluorogenic substrate plus the Chlorella extracts in various concentrations. Subsequently the mixture was incubated for 1 h at 37°C and measured using Flexstation 3 (Molecular Devices, San Jose, CA, USA) at excitation 320 nm and emission 405 nm.

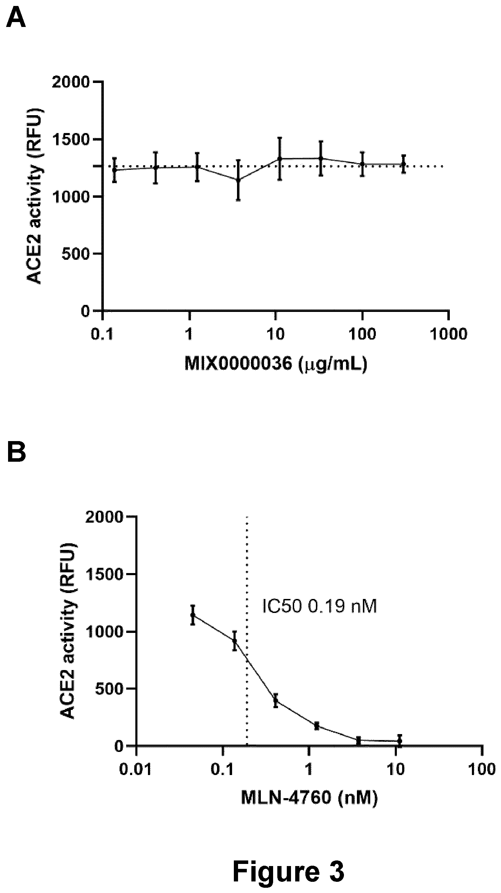

Chlorella extract in the range of 4.5 µg/mL to 10000 µg/mL did not show an effect on ACE2 enzyme activity, indicating that the main function of ACE2, degrading angiotensin II to angiotensin 1-7, is maintained (Figure 3). As positive control, a known ACE2 inhibitor (MLN-4760) in the range of 0.045 nM to 11100 nM was used. More specifically, Figure 3 shows the activity of the catalytic pathway of ACE2, measured by Fluorescence, in the presence of various concentrations of Chlorella extract (MIX0000036, Figure 3A) and MLN-4760 as positive control (Figure 3B), in which RFU are relative fluorescent units. The half maximal inhibitory concentration (IC50) for MLN-4760 was 0.19 nM.

### Experiment 4 - Chlorella extract protects cells from infection against active SARS-CoV-2

To characterize the protective effect of Chlorella extract on the vulnerability of Vero E6 cells to infection by SARS-CoV-2, a Virus-Neutralization-Test was performed that monitors cell survival.

Virus suspensions containing SARS-CoV-2 at a dose of 200 TCID50 were prepared for different concentrations of Chlorella and Remdesivir at a final volume of 200 µl. The resulting virus suspensions were incubated for 1 h at 37° C in a transport box containing CO₂ bags and H₂O.

As per the manufacturer's protocol, Vero E6 cells were grown in MEM (M3303), 1.25% L-Glutamine, 1% P/S, 1% NEAA, Bicarbonat and 10% FCS, (all Bioswisstec, Switzerland) until confluency. Cells were then seeded at 200.000/well in a 96 well plate. Infection of Vero E6 cells was carried out by removing the cell medium from the cell suspensions and adding the prepared 200 µl virus suspensions. Cell suspensions containing Vero E6 together with SARS-CoV-2/Chlorella/Remdesivir were incubated at 37° C for 4 days in a transport box containing CO₂ bags and H₂O. After the incubation, Vero E6 cell viability was measured using the Celltiter Glo (Promega) according to the manufacturer's protocol and measured using a GloMax plate reader (Promega).

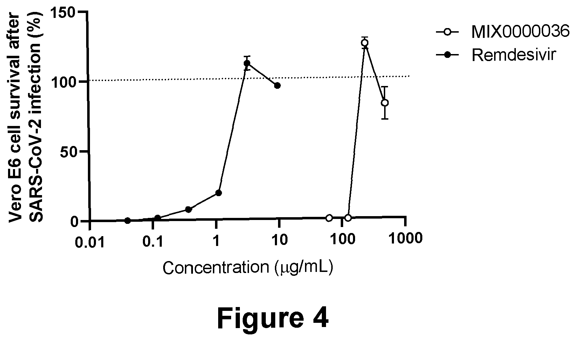

Figure 4 shows the antiviral activity of Chlorella extract (MIX0000036, white circles) using active SARS-CoV-2 (French isolate) and VeroE6 cells. Viral fusion in VeroE6 cells predominantly occurs after ACE2 binding, followed by endocytosis. Pre-treatment of Chlorella extract across the tested concentration of 63 µg/mL to 2000 µg/mL followed by SARS-CoV-2 infection inhibited SARS-CoV-2 viral entry for 72 hours with an IC50 of approximately 140 µg/mL in VeroE6. In the presence of Chlorella extract, the percentage of Vero E6 cell survival after SARS-CoV-2 infection peaked at 250 µg/mL.

As a positive control, the broad-spectrum antiviral medication Remdesivir was used (Figure 4, black circles) which blocked SARS-CoV-2 replication across the tested range of 0.04 µg/mL to 10 µg/mL by arresting viral RNA synthesis.

## Claims

1. An orally inhaled formulation comprising Chlorella or an extract thereof for use in the treatment of a viral disease.

2. The orally inhaled formulation according to claim 1, wherein the orally inhaled formulation is used to stop viral infection via the nasal and oral cavities in a subject by shielding the epithelium cells of the nasal and oral mucosa.

3. The orally inhaled formulation according to claim 1 or 2, for use as a limiter or preventer of viral particles released by the dying cells from colonizing new cells, such as the olfactory and gingival epithelium.

4. The orally inhaled formulation according to any one of the preceding claims , wherein the Chlorella extract comprises one or more of: protein, lipid, vitamin, mineral, folate, iron, fibre, and/or pigment.

5. The orally inhaled formulation according to claim 4, wherein the protein is selected from one or more of Plastocyanin (A0A2P6TGD1), Glycine-rich 2 (A0A2P6TDY2), SMAD FHA domain-containing (A0A2P6U3K7), Thylakoid lumenal kDa chloroplastic (A0A2P6TNZ5), Peptidylprolyl isomerase (A0A2P6TGB0), Calmodulin (A0A2P6TFR8), 10 kDa chaperonin-like (A0A2P6THP7), Membrane isoform A (A0A2P6TEF6), L-inducible nipa (A0A2P6TU12), Beta-lg-H3 fasciclin (A0A2P6TQQ9).

6. The orally inhaled formulation according to claim 4, wherein pigment is chlorophyll.

7. The orally inhaled formulation according to any one of the preceding claims, wherein the viral disease is caused by coronavirus (SARS-CoV-2).

8. The orally inhaled formulation according to any one of the preceding claims, for use as an inhibitor of the binding of the Spike protein receptor binding domain (RBD) to Angiotensin-Converting Enzyme 2 (ACE2).

9. A nasal spray comprising Chlorella or an extract thereof for use in the treatment of a viral disease.

10. A mouth wash comprising Chlorella or an extract thereof for use in the treatment of a viral disease.

11. A composition comprising Chlorella or an extract thereof, wherein the composition is administered as a nasal spray; a mouth wash; or an orally inhaled formulation.

12. The composition according to claim 11, wherein the composition is one or more of a powder, a liquid, an aerosol, a gel, a paste, or a cream.

13. The composition according to claim 11 or 12, wherein the composition comprises at least about 0.001 µg/mL of Chlorella or an extract thereof.

14. The composition according to any one of claims 11 to 13, wherein the Chlorella extract comprises one or more of: protein, lipid, vitamin, mineral, folate, iron, fibre, and/or pigment.

15. The composition according to any one of claims 11 to 14, for use as an inhibitor of the binding of the Spike protein receptor binding domain (RBD) to Angiotensin-Converting Enzyme 2 (ACE2).
